# EUROPÄISCHE PATENTSCHRIFT

(11) **EP 0 805 138 B1**
(45) Veröffentlichungstag und Bekanntmachung des Hinweises auf die Patenterteilung: **28.03.2001**
(21) Anmeldenummer: 97106696.4
(22) Anmeldetag: 23.04.1997
(51) Int. Cl.: C07C 45/50

(54) **Verfahren zur Hydroformylierung olefinisch ungesättigter Verbindungen**
Process for the hydroformylation of olefinic unsaturated compounds
Procédé pour l'hydroformylation de composés à insaturation oléfinique

(30) Priorität: 30.04.1996 DE 19617257
(43) Veröffentlichungstag der Anmeldung: 05.11.1997
(73) Patentinhaber: Celanese Chemicals Europe GmbH, 60439 Frankfurt am Main (DE)
(72) Erfinder: Bahrmann, Helmut, Dr., 46499 Hamminkeln (DE); Frohning, Dieter, Dr., 46485 Wesel (DE); Gick, Wilhelm, Dr., 47199 Duisburg (DE); Höfs, Wolfgang, 46147 Oberhausen (DE); Kalbfell, Heinz, 46514 Schermbeck (DE); Kappesser, Harald, 46149 Oberhausen (DE); Lappe, Peter, Dr., Plano, TX 75025 (US); Schalapski, Kurt, 46147 Oberhausen (DE); Wiebus, Ernst, 46147 Oberhausen (DE); Zgorzelski, Wolfgang, 46149 Oberhausen (DE)

(56) Entgegenhaltungen:
- EP-A- 0 562 451
- EP-A- 0 646 563
- GB-A- 1 387 657
- US-A- 5 367 106

## Beschreibung

Die Erfindung betrifft ein verbessertes Verfahren zur Hydroformylierung olefinisch ungesättigter Verbindungen in Gegenwart einer wäßrigen, wasserlösliche Rhodium-Komplexverbindungen enthaltenden Katalysatorlösung und insbesondere die Nutzung der mit dem Abgas aus der Hydroformylierungszone entweichenden, nicht umgesetzten Olefine.

Die Reaktion von Verbindungen, die olefinische Doppelbindungen enthalten, mit Kohlenmonoxid und Wasserstoff, ist das gängige technische Verfahren zur Herstellung von Aldehyden (Oxosynthese).

Der Prozeß ist nicht auf den Einsatz olefinischer Kohlenwasserstoffe beschränkt, sondern erstreckt sich auch auf Ausgangsstoffe, die außer der Doppelbindung noch funktionelle Gruppen aufweisen, vorwiegend solche, die unter den Reaktionsbedingungen unverändert bleiben.

Die klassische Oxosynthese arbeitet mit Kobalt als Katalysator. Seine Wirksamkeit beruht auf der Bildung von Kobaltcarbonylverbindungen unter der Einwirkung von Wasserstoff und Kohlenmonoxid bei Drücken oberhalb 20 MPa und Temperaturen von etwa 120°C und mehr auf metallisches Kobalt oder Kobaltverbindungen.

In den letzten 30 Jahren wurde Kobalt zunehmend durch Rhodium als Katalysator ersetzt. Das Platinmetall wird als Komplexverbindung eingesetzt, die neben Kohlenmonoxid vorzugsweise Phosphine als Liganden enthält. Rhodium als Katalysator erlaubt es, bei niedrigen Drücken zu arbeiten, überdies erzielt man höhere Ausbeuten und bevorzugt werden die für die Weiterverarbeitung wertvolleren unverzweigten Produkte gebildet, wenn man von geradkettigen endständigen Olefinen ausgeht.

Eine weitere Vervollkommnung der Oxosynthese bedeutet der Übergang von homogen im Reaktionsmedium, d.h. im Einsatzmaterial und im Reaktionsprodukt, gelösten Katalysatoren zu wäßrigen Katalysatorlösungen, die als eigene Phase getrennt von Einsatzstoff und Umsetzungsprodukt vorliegen. Diese Variante der Reaktion ist z.B. in DE-B-26 27 354 beschrieben. Ihr besonderer Vorteil ist die leichte Trennung von Reaktionsprodukt und Katalysator, die schonend, ohne Anwendung thermischer Verfahrensschritte erfolgt und daher Verluste vermeidet, die durch Folgereaktionen der entstandenen Aldehyde eintreten. Weiterhin erzielt man sehr hohe Ausbeuten und bei Verwendung unverzweigter endständiger Olefine erhält man ganz überwiegend n-Aldehyde.

Aus Gründen der Prozeßökonomie, insbesondere um große Reaktoren oder lange Reaktionszeiten zu vermeiden, führt man die Umsetzung nicht bis zum vollständigen Verbrauch der olefinisch ungesättigten Verbindungen durch, sondern begnügt sich häufig mit der Umwandlung von lediglich 60 bis 95 % des Ausgangsmaterials zur gewünschten Endverbindung. Im Abgas, das die Hydroformylierungszone verläßt, befinden sich daher neben überschüssigem Kohlenmonoxid und Wasserstoff nicht umgesetztes olefinisches Einsatzmaterial. Während man früher häufig darauf verzichtete, diese Wertstoffe zurückzugewinnen, ist man heute bemüht, sie möglichst vollständig zu nutzen. Dieses Bestreben führte zur Entwicklung einer Reihe unterschiedlich ausgestalteter Prozesse.

Nach einem bekannten Verfahren (vgl. EP 0 111 257 B1) setzt man Abgas, das aus einer Hydroformylierungsstufe kommt, in der Olefin mit Kohlenmonoxid und Wasserstoff in Gegenwart einer wäßrigen Rhodiumkomplexverbindungen enthaltenden Katalysatorlösung bei niedrigem Druck zur Reaktion gebracht wird, in einer zweiten Stufe nach dem klassischen Oxoverfahren bei hohem Druck und in Gegenwart von Kobaltkatalysatoren um. Dieses Verfahren hat sich in der Praxis sehr gut bewährt, insbesondere wenn die Möglichkeit bestand, eine moderne Hydroformylierungsanlage mit einer bereits vorhandenen, mit Kobalt als Katalysator arbeitenden Anlage, zu kombinieren.

Ein ähnliches zweistufiges Hydroformylierungsverfahren wird in EP-0 562 451 offenbart, das sich von dem aus EP-0 111 257 B1 bekannten Verfahren jedoch dadurch unterscheidet, daß die zweite homogen durchgeführte Hydroformylierungsstufe in Gegenwart von Rhodiumverbindungen erfolgt. Hierbei wird Rhodium dem Reaktionsgemisch als Metall, als Carbonylverbindung oder als Salz einer Carbonsäure zugeführt. Ein Buten-1 und Buten-2 enthaltendes Einsatzgemisch kann mit bis zu 99% des eingesetzten Olefins in n- und i-Valeraldehyd überführt werden.

Die Durchführung eines einstufigen homogenen Hydroformylierungsverfahrens in Gegenwart Rhodium und organische Phosphine enthaltender Katalysatoren ist aus EP-0 417 597 bekannt. Bei der offenbarten Arbeitsweise wird Crotonsäuremethylester bei Drücken von 20 bis 30 MPa in 2-Formylbuttersäuremethylester überführt.

Nach einer anderen Arbeitsweise (vgl. EP 01 88 246 B1) schaltet man zwei Rhodiumkatalysierte Hydroformylierungsstufen hintereinander. In der ersten Stufe wird unter Rückführung von Flüssigkeit oder Gas Olefin, Kohlenmonoxid und Wasserstoff in Gegenwart eines löslichen Rhodium-Phosphor-Komplexkatalysators, freiem Phosphor-Liganden und höhersiedenden Aldehyd-Kondensationsnebenprodukten umgesetzt. Das Abgas, es enthält Olefin, gegebenenfalls Aldehyd, ferner Wasserstoff, Kohlenmonoxid und Alkan-Nebenprodukt, wird einem entkoppelten, d. h. von der ersten Stufe separat betriebenen sekundären Rhodium-katalysierten Hydroformylierungsverfahren zugeleitet, in dem unter Rückführung von Flüssigkeit oder Gas das Abgas zusammen mit zugesetztem Kohlenmonoxid und Wasserstoff zur Reaktion gebracht wird. Dieser Prozeß beschränkt sich darauf, die Hydroformylierung sowohl in der ersten als auch in der zweiten Stufe in Gegenwart eines im Reaktionsgemisch homogen gelösten Katalysators durchzuführen. Die weitgehende Übereinstimmung der Reaktionsbedingungen, insbesondere des Katalysatorsystems in der Haupt- und in der Nachumsetzung schließt mit hoher Sicherheit aus, daß das Abgas der ersten Stufe Komponenten enthält, die die Hydroformylierung in der zweiten Stufe beeinträchtigen könnten.

Die Übertragung der zweistufigen, in homogenen Phasen durchgeführten Umsetzung auf einen Prozeß, der in der ersten und in der zweiten Stufe wäßrige Katalysatorlösungen verwendet, ist mit vertretbarem technischen Aufwand nicht möglich. Sie scheitert daran, daß das Abgas der Primärstufe auf Grund des hohen Umsatzes Olefin in niedriger Konzentration enthält. Seine Verarbeitung in der Sekundärstufe erfordert bei Einsatz eines in Wasser gelösten Katalysatorsystems daher entweder lange Reaktionszeiten oder besondere technische Vorkehrungen um die Reaktionszeit zu beschränken und dennoch einen hohen Umsatz zu erzielen.

Es bestand somit die Aufgabe, einen Prozeß zu entwickeln, der es unter ökonomisch vertretbaren Bedingungen erlaubt, olefinische Verbindungen, die im Abgas einer mit wäßriger Katalysatorlösung durchgeführten Hydroformylierungsreaktion enthalten sind, zu Carbonylverbindungen umzusetzen.

Die Erfindung besteht in einem Verfahren zur Hydroformylierung olefinisch ungesättigter Verbindungen, wobei die Reaktion in einer ersten Reaktionsstufe in einem heterogenen Reaktionssystem unter Verwendung einer wäßrigen Lösung, wasserlösliche organische Phosphor(III)-verbindungen in komplexer Bindung enthaltender Rhodiumverbindungen als Katalysatoren bei Drücken von 0,4 bis 10 MPa erfolgt und Abgas gebildet wird und das Abgas in der ersten Reaktionsstufe einer zweiten Reaktionsstufe zugeführt wird, in der im Abgas noch vorhandene Restmengen der olefinisch ungesättigten Verbindungen in einem homogenen Reaktionssystem umgesetzt werden. Es ist dadurch gekennzeichnet, daß die Reaktion in der zweiten Reaktionsstufe in Gegenwart von Komplexverbindungen des Rhodiums mit organischen Phosphor(lll)-verbindungen als Katalysatoren bei Drücken von 15 bis 40 MPa erfolgt.

Das neue Verfahren stellt sicher, daß der größte Teil der im Abgas enthaltenen, in der ersten Stufe nicht umgesetzten olefinischen Verbindungen hydroformyliert wird. Auf diese Weise lassen sich, bezogen auf den Gesamtprozeß, häufig mehr als 98 % der eingesetzten Ausgangsstoffe in die gewünschten Carbonylverbindungen überführen, wobei der Umsatz im wesentlichen von der Art des Einsatzmaterials und den Reaktionsbedingungen in den beiden Stufen abhängt. Besonders hervorzuheben ist, daß nicht oder kaum verwertbare Nebenprodukte nur in ganz untergeordneter Menge gebildet werden. Die hohe Effizienz des erfindungsgemäßen Prozesses war nicht vorauszusehen. Hierbei ist u.a. zu berücksichtigen, daß die olefinischen Verbindungen im Abgas in beträchtlicher Verdünnung vorliegen. So beträgt z.B. bei der Hydroformylierung niedriger Olefine der Anteil des nicht umgesetzten olefinischen Einsatzmaterials im Abgas nur etwa 20 bis 50 Gew.-%. Derartige Konzentrationen stehen bei dieser Reaktionsart einer weitgehenden Umsetzung der ungesättigten Ausgangsverbindung entgegen. Die mit dem Abgas aus der ersten Reaktionsstufe ausgetragenen Verunreinigungen, hierbei handelt es sich u.a. um Abbauprodukte des Katalysatorsystems wie Mercaptane und um Wasser, dem Lösungsmittel für den Katalysator in der ersten Stufe, beeinträchtigen überraschenderweise nicht die Wirksamkeit des Katalysators in der zweiten Stufe. Ein solches Verhalten war nicht zu erwarten, weil insbesondere organische Derivate des Schwefelwasserstoffs als Katalysatorgifte bekannt sind. Hierbei ist zu berücksichtigen, daß Mercaptane in Wasser kaum löslich sind, infolgedessen nicht in die Katalysatorlösung der ersten Reaktionsstufe gelangen und daher den Katalysator auch nicht schädigen können. Dagegen reichern sich die Mercaptane aufrund ihrer guten Löslichkeit in organischen Medien im homogenen, Katalysator enthaltenden, Reaktionsgemisch der zweiten Reaktionsstufe an. Darüber hinaus bildet auch Wasser mit Rhodium Komplexverbindungen und vermag daher die organischen Phosphorliganden in den homogen gelösten Rhodium-Komplexverbindungen zumindest teilweise unter Bildung katalytisch inaktiver Substanzen zu ersetzen.

Die erste Reaktionsstufe des neuen Verfahrens führt man als heterogene Reaktion in einem Zweiphasensystem durch, eine Umsetzung, die z.B. in der DE-B-26 27 354 beschrieben ist. Dieser Prozeß ist charakterisiert durch das Vorliegen einer organischen Phase, die das olefinische Ausgangsmaterial und das Reaktionsprodukt enthält und einer wäßrigen Phase, in der der Katalysator gelöst ist. Als Katalysatoren werden wasserlösliche Rhodium-Komplexverbindungen eingesetzt, die wasserlösliche organische Phosphor(III)-verbindungen als Liganden enthalten. Beispiele für wasserlösliche Phosphor(III)-verbindungen, die mit Rhodium Komplexverbindungen bilden, sind Triarylphosphine, Trialkylphosphine und arylierte bzw. alkylierte Diphosphine, deren organische Reste Sulfonsäuregruppen oder Carboxylgruppen enthalten. Ihre Herstellung und Anwendung ist z.B. aus DE-B 26 27 354, EP 0 103 810 B1, EP 0 163 234 B1 und EP 0 571 819 A1 bekannt. Weitere Gruppen geeigneter Verbindungen sind sulfonierte oder carboxylierte organische Phosphite sowie heterocyclische Verbindungen des dreibindigen Phosphors (vgl. z.B. EP 0 575 785 A1, EP 0 646 588 A1).

Die Bedingungen, unter denen die Umsetzung in der ersten Reaktionsstufe abläuft, können innerhalb weiter Grenzen variiert und den individuellen Gegebenheiten angepaßt werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 50 bis 180°C durch. Bevorzugt hält man Temperaturen von 80 bis 140 und insbesondere von 100 bis 130°C ein. Der Gesamtdruck erstreckt sich über einen Bereich von 0,4 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1 : 10 bis 10 : 1, Mischungen, die Wasserstoff und Kohlenmonoxid im Verhältnis 3 : 1 bis 1 : 3 und insbesondere etwa 1 : 1, enthalten, sind besonders geeignet.

Die Rhodium-Konzentration beträgt 20 bis 1000 Gew.-ppm, vorzugsweise 50 bis 500 Gew.-ppm und insbesondere 100 bis 300 Gew.-ppm, jeweils bezogen auf die wäßrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphorliganden, d.h. Ligand, der mit Rhodium keine komplexe Bindung eingegangen ist. Je mol Rhodium wendet man bevorzugt 3 bis 200 mol Phosphor in Form einer wasserlöslichen organischen Phosphorverbindung an. Besonders bewährt haben sich molare Verhältnisse von Rhodium zu Phosphor im Bereich von 1 : 50 bis 1 : 100. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphorliganden unterscheiden. Ebenso kann der in der wäßrigen Katalysatorlösung enthaltene freie Phosphorligand aus einem Gemisch unterschiedlicher wasserlöslicher organischer Phosphorverbindungen zusammengesetzt sein. Man bildet den Katalysator üblicherweise aus den Komponenten Rhodium oder Rhodiumverbindung, organische Phosphorverbindung und Synthesegas unter den Bedingungen der Hydroformylierungsreaktion im Reaktionsgemisch. Er kann der Reaktionsstufe aber auch präformiert, d.h. gesondert hergestellt, zugeführt werden.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wäßrigen Katalysatorphase ist in der EP 0 103 810 B1 beschrieben. Es hat sich als zweckmäßig erwiesen, die Katalysatorlösung im Kreis zu führen und gegebenenfalls auftretende Katalysatorverluste durch Zufuhr von Frischkatalysator auszugleichen.

Um den Umsatz je Zeiteinheit von olefinisch ungesättigten Verbindungen, die in der wäßrigen Katalysatorlösung nur wenig löslich sind, zu erhöhen, kann es sich empfehlen, dieser Lösung ein Phasentransferreagenz (Lösungsvermittler) zuzusetzen. Es verändert die physikalischen Eigenschaften der Grenzflächen zwischen den beiden flüssigen Phasen und erleichtert den Übergang des organischen Reaktanten in die wäßrige Katalysatorphase.

Als Lösungsvermittler sind Verbindungen bekannt, deren hydrophile Gruppen ionisch (anionisch oder kationisch) oder nichtionisch sind. Zu den anionenaktiven Verbindungen gehören Natrium-, Kalium- oder Ammoniumsalze von Carbonsäuren, vorzugsweise solchen mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wäßriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. in EP 0 157 316 B1 beschrieben.

Schließlich können auch Rhodiumkomplexverbindungen eingesetzt werden, die gleichzeitig Katalysator und Phasentransferreagenz sind. Eine solche Arbeitsweise ist z.B. Gegenstand der EP 0 163 234 B1.

Üblicherweise strebt man in der ersten Reaktionsstufe einen möglichst weitgehenden Umsatz der olefinisch ungesättigten Verbindungen an. In Einzelfällen kann jedoch auch auf einen mehr oder minder großen Teilumsatz hin gearbeitet werden.

Das aus der ersten Reaktionsstufe entweichende Abgas (Abgasstom) setzt sich zusammen aus dem Abgas, das dem Reaktor unmittelbar entnommen wird (Reaktorabgas), um eine Anreicherung von Inerten in dem, im Kreis geführten Gasgemisch zu vermeiden und den gasförmigen Anteilen, die bei der Trennung von Katalysatorlösung und rohem Reaktionsprodukt im Phasentrenner auftreten (Produktabgas). Der Abgasstrom besteht im wesentlichen aus nicht umgesetzter olefinischer Verbindung, Kohlenmonoxid, Kohlendioxid, Wasserstoff und den Hydrierungsprodukten des Olefins. Dieses Gasgemisch wird ohne weitere Zwischenbehandlung, insbesondere ohne Reinigung, jedoch gegebenenfalls nach Zumischen von Wasserstoff allein oder im Gemisch mit Kohlenmonoxid als Einsatzmaterial einer zweiten Hydroformylierungsstufe aufgegeben.

Die zweite Reaktionsstufe wird unabhängig von der ersten betrieben. In ihr werden die im Abgasstrom vorhandenen Restmengen der olefinisch ungesättigten Verbindungen in einem homogenen Reaktionssystem mit Kohlenmonoxid und Wasserstoff umgesetzt. Der Begriff homogenes Reaktionssystem steht für eine im wesentlichen aus Lösungsmittel, Katalysator, olefinisch ungesättigter Verbindung und Reaktionsprodukt zusammengesetzte homogene Lösung. Als Katalysatoren werden Rhodium-Komplexverbindungen verwendet, die organische Phosphor(III)-verbindungen als Liganden enthalten. Derartige Komplexverbindungen und ihre Herstellung sind bekannt (vgl. z. B. US 3 527 809 A1, US 4 148 830 A1, US 4 247 486 A1, US 4 283 562 A1). Sie können als einheitliche Komplexverbindung oder auch als Gemisch unterschiedlicher Komplexverbindungen eingesetzt werden. Die Rhodium-Konzentration im Reaktionsmedium erstreckt sich über einen Bereich von etwa 1 bis etwa 1000 Gew.-ppm und beträgt vorzugsweise 10 bis 700 Gew.- ppm. Insbesondere wendet man Rhodium in Konzentrationen von 25 bis 500 Gew.-ppm, jeweils bezogen auf das homogene Reaktionsgemisch, an. Wie in der ersten Stufe kann als Katalysator die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigem Phosphorliganden durchzuführen, der mit Rhodium keine Komplexverbindung mehr eingeht. Der freie Phosphorligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US 3 527 809 A1 beschrieben. Zu den bevorzugten Liganden in den Rhodium-Komplexkatalysatoren zählen z.B. Triarylphosphine wie Triphenylphosphin, Trialkylphosphine wie Tri(n-octyl)phosphin, Trilaurylphosphin, Tri(cyclohexyl)phosphin, Alkylphenylphosphine, Cycloalkylphenylphosphine und organische Diphosphite. Wegen seiner leichten Zugänglichkeit wird Triphenylphosphin besonders häufig angewandt.

Üblicherweise beträgt das molare Verhältnis von Rhodium zu Phosphor 1 : 1 bis 1 : 300, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 3 bis 1 : 200 ein. Bei Anwendung von Triarylphosphinen haben sich insbesondere Rh/P-Molverhältnisse von 1 : 50 bis 1 150 bewährt. Werden Trialkylphosphine als Liganden eingesetzt, so beträgt das Molverhältnis von Rhodium zu Phosphor bevorzugt 1 : 3 bis 1 : 20.

Die Hydroformylierungsreaktion wird in Gegenwart eines Lösungsmittels ausgeführt. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe wie Benzol, Toluol oder die Xylole. Andere gebräuchliche Lösungsmittel sind Paraffinöl, Ketone oder Ether. Als besonders geeignete Lösungsmittel haben sich die höhersiedenden Kondensationsverbindungen der Aldehyde erwiesen, die als Nebenprodukte bei der Hydroformylierung entstehen. Der Anteil des Lösungsmittels im Reaktionsmedium kann über einen weiten Konzentrationsbereich variiert werden und beträgt üblicherweise zwischen 20 und 90 Gew.-%, vorzugsweise 50 bis 80 Gew.-%, bezogen auf das Reaktionsgemisch.

Der Reaktionsdruck in der 2. Stufe des Gesamtprozesses liegt im Bereich von 15 bis 40 MPa. Besonders bewährt hat es sich, Drücke zwischen 15 und 35 MPa, vorzugsweise 20 bis 30 MPa einzuhalten. Derartige Bereiche sind für Hydroformylierungen in homogenen Reaktionssystemen und in Gegenwart von Komplexverbindungen des Rhodiums mit organischen Phosphorverbindungen ungewöhnlich, unabhängig davon, ob die Umsetzung ein- oder mehrstufig erfolgt. Das Volumenverhältnis von Wasserstoff zu Kohlenmonoxid beträgt 1 : 10 bis 10 : 1, vorzugsweise 1 : 3 bis 3 : 1 und insbesondere 1 : 1.

Die Reaktionstemperaturen betragen in der zweiten Stufe des neuen Prozesses 50 bis 160°C. Temperaturen von 60 bis 150°C und insbesondere 75 bis 140°C werden bevorzugt.

Wie bereits erwähnt, wird das Reaktionsprodukt der ersten Reaktionsstufe in einem Phasentrenner von der wäßrigen Katalysatorlösung, die in den Prozeß zurückgeführt wird, getrennt. Nach einer bewährten Ausführungsform führt man den Rohaldehyd in einer Stripp-Kolonne im Gegenstrom zu frischem Synthesegas. Hierbei wird Wärme vom Aldehyd auf das Synthesegas übertragen und die im Aldehyd gelöste olefinische Verbindung aus dem Rohprodukt ausgetrieben und zusammen mit dem erwärmten Synthesegas erneut der Reaktion zugeleitet. Das Umsetzungsprodukt der zweiten Reaktionsstufe wird vom Katalysator abdestilliert. Es kann mit dem Produkt der ersten Stufe vereinigt und weiter verarbeitet, z.B. destilliert, werden. Der nach Abtrennung des Aldehyds verbleibende, Katalysator enthaltende Destillationsrückstand der zweiten Reaktionsstufe, wird gegebenenfalls nach Zusatz von Frischkatalysator und Entnahme eines Teils der im Verlauf der Reaktion gebildeten Aldehyd-Kondensationsprodukte in die Reaktionszone rezirkuliert.

Die Umsetzung geradkettiger endständiger olefinisch ungesättigter Verbindungen in der zweiten Reaktionsstufe, d.h. mit homogen im Reaktionsmedium gelöstem Katalysator, ergibt als Reaktionsprodukt ein Aldehydgemisch, das einen höheren Anteil Isoverbindung enthält als das Produkt der ersten Stufe, d.h. der Reaktion unter Verwendung einer heterogenen Katalysatorphase. Daher ist es nach dem neuen Verfahren möglich, durch Wahl des Olefinumsatzes in der ersten Stufe die Anteile n- und iso-Verbindung im Reaktionsprodukt über den Gesamtprozeß den aktuellen Anforderungen anzupassen. Nach einer weiteren Ausführungsform des erfindungsgemäßen Verfahrens kann das Verhältnis von n- und iso-Verbindung im Gesamtprozeß auch durch Zusatz von Olefin zum Abgasgemisch beeinflußt werden, das der zweiten Reaktionsstufe zugeführt wird. Unabhängig von der Art der olefinisch ungesättigten Einsatzstoffe ist die Bildung höhermolekularer Aldehydfolgeprodukte (Dicköl) in der Sekundärstufe sehr gering.

Das erfindungsgemäße Verfahren kann auf olefinisch ungesättigte Verbindungen beliebiger Struktur angewandt werden. Dementsprechend sind als Ausgangsmaterial geeignet sowohl Olefine mit innenständiger als auch mit endständiger Doppelbindung und ebenso geradkettige oder verzweigte Olefine. Überdies können die Olefine auch noch durch funktionelle Gruppen substituiert sein, insbesondere solche, die im Verlauf der Reaktion nicht verändert werden. Auch mehrfach olefinisch ungesättigte Verbindungen kommen als Einsatzstoffe in Betracht. Besonders bewährt hat sich das Verfahren bei der Hydroformylierung olefinisch ungesättiger Kohlenwasserstoffe mit 3 bis 6 Kohlenstoffatomen im Molekül, vorzugsweise Propylen und die isomeren Butene.

Die Erfindung wird in den nachfolgenden Beispielen näher erläutert, jedoch nicht auf die beschriebenen Ausführungsformen beschränkt.

### Beispiel 1

### 1. Reaktionsstufe

In einem Reaktor wird unter intensivem Rühren bei einem Synthesegasdruck (CO : H₂ = 1 : 1) von 5 MPa und bei einer Temperatur von 122°C aus Rhodiumacetat und Triphenylphosphintrisulfonat-Na (TPPTS) der aktive, wasserlösliche Katalysator HRhCO(TPPTS)₃ hergestellt. Rhodiumverbindung und TPPTS werden in einer solchen Menge eingesetzt, daß die Rhodiumkonzentration in der wäßrigen Katalysatorlösung 300 Gew.-ppm und das Molverhältnis von Rhodium zu Phosphor etwa 1 : 100 beträgt.

Über einen Verteilerring am Boden des Reaktors leitet man vorgewärmtes Propylen in die Reaktionszone und setzt dort das Olefin mit Kohlenmonoxid und Wasserstoff bei 122°C und 5 MPa um. Der aus gasförmigen und flüssigen Bestandteilen bestehende Produktstrom wird dem oberen Teil des Reaktors entnommen und einem Phasentrenner zugeführt, in dem die Trennung der wäßrigen Katalysatorlösung vom rohen organischen Reaktionsprodukt und vom Produktabgas erfolgt. Das Produktabgas wird mit dem Reaktorabgas zum Abgasstrom vereinigt. Reaktorabgas wird dem Reaktor entnommen, um eine Anreicherung von Inerten im Gasgemisch zu vermeiden, das im Kreis geführt wird. Bei weiterer Abkühlung scheidet sich aus dem Abgasstrom ein flüssiges Kondensat (Abgaskondensat) ab, das im wesentlichen aus Propylen und Propan besteht.

Die im Phasentrenner anfallende Katalysatorlösung wird in den Reaktor zurückgepumpt. Den Rohaldehyd führt man in einer nachgeschalteten Strippkolonne im Gegenstrom zu Synthesegas. Hierbei geht Wärme vom Aldehyd auf das Synthesegas über und gleichzeitig trägt der Synthesegasstrom im Rohaldehyd gelöstes Propylen und Propan aus. Durch die Abkühlung scheidet sich aus dem Aldehyd Wasser aus, das in den Katalysatorkreislauf zurückgeführt wird. Der Propylenumsatz liegt, je nach Durchsatz und Reinheit des Olefins, unter den gewählten Reaktionsbedingungen zwischen 81 und 91 %. Das Verhältnis von n-Aldehyd zu i-Aldehyd beträgt etwa 20 : 1.

### 2. Reaktionsstufe

Abgasstrom und Abgaskondensat aus der ersten Reaktionsstufe werden gegebenenfalls durch Wasserstoff und/oder Kohlenmonoxid und/oder Propylen ergänzt und zusammen mit der Katalysatorlösung auf 21 MPa komprimiert und dem zweiten Reaktor zugeführt. Der Katalysator besteht aus höhersiedenden Aldehydkondensationsprodukten, in denen Triphenylphosphin (TPP) und die Rhodium-Komplexverbindung HRhCO(TPP)₃ gelöst sind. Das Einsatzgemisch wird mit einer Raumgeschwindigkeit von 0,5 V/V . h vom Boden her in den Reaktor eingeführt. Es hat etwa folgende Zusammensetzung (alle Angaben in Gew.-%).

| | |
|---|---|
| Wasserstoff | 2,20 |
| Kohlenmonoxid | 29,94 |
| Kohlendioxid | 0,31 |
| Inerte | 1,29 |
| Propylen | 40,01 |
| Propan | 10,19 |
| n-Butyraldehyd | 6,98 |
| Isobutyraldehyd | 0,54 |
| Butanole | 0,77 |
| C8-Komponenten | 1,60 |
| > C8-Komponenten | 2,76 |
| Triphenylphosphin | 1,21 |
| Triphenylphosphinoxid | 0,50 |
| Wasser | 1,69 |
| Schwefel | Spuren |

Das Molverhältnis von Phosphor zu Rhodium beträgt etwa 80 : 1. Die Umsetzung der Reaktanten erfolgt bei 132°C. 99 % des eingesetzten Propylens (d.h. des nicht umgesetzten Propylens der ersten Reaktionsstufe und gegebenenfalls dem Abgasstrom vor Eintritt in den zweiten Reaktor zugesetztes Propylen) werden in Aldehyd umgewandelt. Der den zweiten Reaktor verlassende Produktstrom wird über einen Abscheider entspannt. Neben einer flüssigen Phase, dem Aldehyd-Rohprodukt, erhält man eine gasförmige Phase, das Entspannungsgas, das man zur Abscheidung restlichen Aldehyds fraktioniert kondensiert.

Der Rohaldehyd wird in einer ersten Kolonne vom Katalysator abdestilliert und in einer zweiten Kolonne in n- und i-Butyraldehyd getrennt. Der Katalysator, er fällt als flüssiger Rückstand in der ersten Kolonne an, wird zum überwiegenden Teil in den Reaktor zurückgeführt. Lediglich ein kleiner Teilstrom wird in einer solchen Menge abgetrennt, daß die Konzentration der als Lösungsmittel für den Rhodiumkatalysator verwendeten höhersiedenden Aldehydkondensationsprodukte im zweiten Reaktor etwa konstant bleibt.

Unter den gewählten Reaktionsbedingungen beträgt das Verhältnis von n-Aldehyd : i-Aldehyd 65 : 35.

### Beispiel 2

### 1. Reaktionsstufe

Die Umsetzung der Reaktanten in der ersten Reaktionsstufe erfolgt in der gleichen Weise wie in Beispiel 1 beschrieben.

### 2. Reaktionsstufe

Abgasstrom und Abgaskondensat werden gegebenenfalls durch Wasserstoff und/oder Kohlenmonoxid und/oder Propylen ergänzt und bei 25 bis 27 MPa Druck und einer Temperatur von 130°C in einem Autoklaven umgesetzt. Je 1.500 g Propylen werden 80 mg Rhodium und wechselnde Mengen Trilaurylphosphin eingesetzt, so daß das Molverhältnis von Rhodium zu Phosphin 1 : 5 bzw. 1 : 10 beträgt.

Die Ergebnisse sind in der nachfolgenden Tabelle zusammengestellt. Sie werden mit den Ergebnissen verglichen, die man bie Einsatz von Rh/TPP als Katalysator erhält.

**Tabelle**

| | Trilaurylphosphin | | Triphenylphosphin |
|---|---|---|---|
| Rh/P-Verhältnis | 1 : 5 | 1 : 10 | 1 : 10 |
| Umsatz (%) | 96 | 98 | 95 |
| n-/iso-Verhältnis | 64/36 | 64/36 | 61/39 |

## Patentansprüche

1. Verfahren zur Hydroformylierung olefinisch ungesättigter Verbindungen, wobei die Reaktion in einer ersten Reaktionsstufe in einem heterogenen Reaktionssystem unter Verwendung einer wäßrigen Lösung, wasserlösliche organische Phosphor(lll)-verbindungen in komplexer Bindung enthaltender Rhodiumverbindungen als Katalysatoren bei Drücken von 0,4 bis 10 MPa erfolgt und Abgas gebildet wird und das Abgas der ersten Reaktionsstufe einer zweiten Reaktionsstufe zugeführt wird, in der im Abgas noch vorhandene Restmengen der olefinisch ungesättigten Verbindungen in einem homogenen Reaktionssystem umgesetzt werden, dadurch gekennzeichnet, daß die Reaktion in der zweiten Reaktionsstufe in Gegenwart von Komplexverbindungen des Rhodiums mit organischen Phosphor(III)-verbindungen als Katalysatoren bei Drücken von 15 bis 40 MPa erfolgt.

2. Verfahren nach Anspruch 1, dadurch gekennzeichnet, daß die Hydroformylierung in der ersten Reaktionsstufe bei Temperaturen von 50 bis 180°C und einer Rhodiumkonzentration von 20 bis 1000 Gew.-ppm, bezogen auf die wäßrige Katalysatorlösung, erfolgt und das molare Verhältnis von Rhodium zu Phosphor in der Katalysatorlösung 1 : 3 bis 1 : 200 beträgt.

3. Verfahren nach Anspruch 1 oder 2, dadurch gekennzeichnet, daß der Druck in der ersten Reaktionsstufe 1 bis 6 und insbesondere 1,5 bis 5 MPa beträgt.

4. Verfahren nach einem oder mehreren der Ansprüche 1 bis 3, dadurch gekennzeichnet, daß die Temperatur in der ersten Reaktionsstufe 80 bis 140 und insbesondere 100 bis 130°C beträgt.

5. Verfahren nach einem oder mehreren der Ansprüche 1 bis 4, dadurch gekennzeichnet, daß die Konzentration des Rhodiums in der wäßrigen Katalysatorlösung 50 bis 500 und insbesondere 100 bis 300 Gew.-ppm beträgt.

6. Verfahren nach einem oder mehreren der Ansprüche 1 bis 5, dadurch gekennzeichnet, daß das molare Verhältnis von Rhodium zu Phosphor in der wäßrigen Katalysatorlösung 1 : 50 bis 1 : 100 beträgt.

7. Verfahren nach einem oder mehreren der Ansprüche 1 bis 6, dadurch gekennzeichnet, daß als wasserlösliche organische Phosphor(III)-verbindungen sulfonierte oder carboxylierte aliphatische, aromatische oder gemischt aliphatisch-aromatische Phosphine oder Phosphite eingesetzt werden.

8. Verfahren nach einem oder mehreren der Ansprüche 1 bis 7, dadurch gekennzeichnet, daß die Hydroformylierung in der zweiten Reaktionsstufe in Gegenwart eines Lösungsmittels bei Temperaturen von 50 bis 160°C und einer Rhodiumkonzentration von 1 bis 1000 Gew.-ppm, bezogen auf das homogene Reaktionsgemisch, erfolgt und das molare Verhältnis von Rhodium zu Phosphor im Reaktionsgemisch 1 : 1 bis 1 : 300 beträgt.

9. Verfahren nach Anspruch 8, dadurch gekennzeichnet, daß der Druck in der zweiten Reaktionsstufe 15 bis 35 und insbesondere 20 bis 30 MPa beträgt.

10. Verfahren nach Anspruch 8 oder 9, dadurch gekennzeichnet, daß die Temperatur in der zweiten Reaktionsstufe 60 bis 150°C und insbesondere 75 bis 140°C beträgt.

11. Verfahren nach einem oder mehreren der Ansprüche 8 bis 10, dadurch gekennzeichnet, daß die Rhodiumkonzentration in der zweiten Reaktionsstufe 10 bis 700 Gew.-ppm und insbesondere 25 bis 500 Gew.-ppm, bezogen auf das Reaktionsgemisch, beträgt.

12. Verfahren nach einem oder mehreren der Ansprüche 8 bis 11, dadurch gekennzeichnet, daß in der zweiten Reaktionsstufe das molare Verhältnis von Rhodium zu Phosphor im Reaktionsgemisch 1 : 3 bis 1 : 200 beträgt.

13. Verfahren nach einem oder mehreren der Ansprüche 8 bis 12, dadurch gekennzeichnet, daß als organische Phosphor(III)-verbindungen aliphatische, aromatische oder gemischt aliphatisch-aromatische Phosphine oder Phosphite eingesetzt werden.

14. Verfahren nach Anspruch 13, dadurch gekennzeichnet, daß die organische Phosphor(III)-verbindung ein Triarylphosphin ist und das molare Verhältnis von Rhodium zu Phosphor im Reaktionsgemisch 1 : 50 bis 1 : 150 beträgt.

15. Verfahren nach Anspruch 14, dadurch gekennzeichnet, daß das Triarylphosphin Triphenylphosphin ist.

16. Verfahren nach Anspruch 13, dadurch gekennzeichnet, daß die organische Phosphor(III)-verbindung ein Trialkylphosphin ist und das molare Verhältnis von Rhodium zu Phosphor im Reaktionsgemisch 1 : 3 bis 1 : 20 beträgt.

17. Verfahren nach Anspruch 16, dadurch gekennzeichnet, daß das Trialkylphosphin Trilaurylphosphin ist.

## Claims

1. A process for the hydroformylation of olefinically unsaturated compounds, in which the reaction in a first reaction stage is carried out in a heterogeneous reaction system using an aqueous solution comprising, as catalysts, rhodium compounds containing water-soluble organic phosphorus(III) compounds in complexed form at pressures of from 0.4 to 10 MPa and waste gas is formed, and the waste gas from the first reaction stage is fed to a second reaction stage in which the residual amounts of the olefinically unsaturated compounds still present in the waste gas are reacted in a homogeneous reaction system, wherein the reaction in the second reaction stage is carried out in the presence of, as catalysts, rhodium complexes of organic phosphorus(III) compounds at pressures of from 15 to 40 MPa.

2. The process as claimed in claim 1, wherein the hydroformylation in the first reaction stage is carried out at temperatures of from 50 to 180°C and a rhodium concentration of from 20 to 1000 ppm by weight, based on the aqueous catalyst solution, and the molar ratio of rhodium to phosphorus in the catalyst solution is from 1:3 to 1:200.

3. The process as claimed in claim 1 or 2, wherein the pressure in the first reaction stage is from 1 to 6 MPa and in particular from 1.5 to 5 MPa.

4. The process as claimed in one or more of claims 1 to 3, wherein the temperature in the first reaction stage is from 80 to 140°C and in particular from 100 to 130°C.

5. The process as claimed in one or more of claims 1 to 4, wherein the concentration of the rhodium in the aqueous catalyst solution is from 50 to 500 ppm by weight and in particular from 100 to 300 ppm by weight.

6. The process as claimed in one or more of claims 1 to 5, wherein the molar ratio of rhodium to phosphorus in the aqueous catalyst solution is from 1:50 to 1:100.

7. The process as claimed in one or more of claims 1 to 6, wherein the water-soluble organic phosphorus(III) compounds used are sulfonated or carboxylated aliphatic, aromatic or mixed aliphatic-aromatic phosphines or phosphites.

8. The process as claimed in one or more of claims 1 to 7, wherein the hydroformylation in the second reaction stage is carried out in the presence of a solvent at temperatures of from 50 to 160°C and a rhodium concentration of from 1 to 1000 ppm by weight, based on the homogeneous reaction mixture, and the molar ratio of rhodium to phosphorus in the reaction mixture is from 1:1 to 1:300.

9. The process as claimed in claim 8, wherein the pressure in the second reaction stage is from 15 to 35 MPa and in particular from 20 to 30 MPa.

10. The process as claimed in claim 8 or 9, wherein the temperature in the second reaction stage is from 60 to 150°C and in particular from 75 to 140°C.

11. The process as claimed in one or more of claims 8 to 10, wherein the rhodium concentration in the second reaction stage is from 10 to 700 ppm by weight and in particular from 25 to 500 ppm by weight, based on the reaction mixture.

12. The process as claimed in one or more of claims 8 to 11, wherein, in the second reaction stage, the molar ratio of rhodium to phosphorus in the reaction mixture is from 1:3 to 1:200.

13. The process as claimed in one or more of claims 8 to 12, wherein the organic phosphorus(III) compounds used are aliphatic, aromatic or mixed aliphatic-aromatic phosphines or phosphites.

14. The process as claimed in claim 13, wherein the organic phosphorus(III) compound used is a triarylphosphine and the molar ratio of rhodium to phosphorus in the reaction mixture is from 1:50 to 1:150.

15. The process as claimed in claim 14, wherein the triarylphosphine is triphenylphosphine.

16. The process as claimed in claim 13, wherein the organic phosphorus(III) compound is a trialkylphosphine and the molar ratio of rhodium to phosphorus in the reaction mixture is from 1:3 to 1:20.

17. The process as claimed in claim 16, wherein the trialkylphosphine is trilaurylphosphine.

## Revendications

1. Procédé pour l'hydroformylation de composés oléfiniquement insaturés, la réaction étant réalisée dans une première étape de réaction dans un système réactionnel hétérogène en utilisant comme catalyseur une solution aqueuse de composés du rhodium contenant dans une liaison complexe des composés organiques solubles dans l'eau du phosphore (III) à des pressions de 0,4 à 10 MPa et des effluents gazeux étant formés et les effluents gazeux de la première étape de réaction étant introduits dans une seconde étape de réaction dans laquelle les quantités résiduelles des composés oléfiniquement insaturés encore présents dans les effluents gazeux réagissent dans un système réactionnel homogène, caractérisé en ce que la réaction est réalisée dans la seconde étape de réaction en présence de composés complexes du rhodium avec des composés organiques du phosphore (III) comme catalyseurs à des pressions de 15 à 40 MPa.

2. Procédé selon la revendication 1, caractérisé en ce que l'hydroformylation est réalisée dans la première étape de réaction à des températures de 50 à 180°C et à une concentration en rhodium de 20 à 1 000 ppm en poids, rapportées à la solution aqueuse de catalyseur et le rapport molaire du rhodium au phosphore dans la solution de catalyseur est de 1:3 à 1:200.

3. Procédé selon la revendication 1 ou 2, caractérisé en ce que la pression dans la première étape de réaction est comprise entre 1 et 6 et en particulier entre 1,5 et MPa.

4. Procédé selon l'une ou plusieurs quelconques des revendications 1 à 3, caractérisé en ce que la température dans la première étape de réaction est de 80 à 140 et en particulier de 100 à 130°C.

5. Procédé selon l'une ou plusieurs quelconques des revendications 1 à 4, caractérisé en ce que la concentration du rhodium dans la solution aqueuse de catalyseur est de 50 à 500 et en particulier de 100 à 300 ppm en poids.

6. Procédé selon l'une ou plusieurs quelconques des revendications 1 à 5, caractérisé en ce que le rapport molaire du rhodium au phosphore dans la solution aqueuse de catalyseur est de 1:50 à 1:100.

7. Procédé selon l'une ou plusieurs quelconques des revendications 1 à 6, caractérisé en ce que l'on utilise comme composés organiques solubles dans l'eau du phosphore (III) des phosphines ou des phosphites aliphatiques, aromatiques ou mélangées aliphatiques-aromatiques sulfonées ou carboxylées.

8. Procédé selon l'une ou plusieurs quelconques des revendications 1 à 7, caractérisé en ce que l'hydroformylation est réalisée dans la seconde étape de réaction en présence d'un solvant à des températures de 50 à 160°C et à une concentration en rhodium de 1 à 1 000 ppm en poids, rapportées au mélange réactionnel homogène, et le rapport molaire du rhodium au phosphore dans le mélange réactionnel est de 1:1 à 1:300.

9. Procédé selon la revendication 8, caractérisé en ce que la pression dans la seconde étape de réaction est de 15 à 35 et en particulier de 20 à 30 MPa.

10. Procédé selon la revendication 8 ou 9, caractérisé en ce que la température dans la seconde étape de réaction est de 60 à 150°C et en particulier de 75 à 140°C.

11. Procédé selon l'une ou plusieurs quelconques des revendications 8 à 10, caractérisé en ce que la concentration en rhodium dans la seconde étape de réaction est de 10 à 700 ppm en poids et en particulier de 25 à 500 ppm en poids, rapportées au mélange réactionnel.

12. Procédé selon l'une ou plusieurs quelconques des revendications 8 à 11, caractérisé en ce que le rapport molaire du rhodium au phosphore dans le mélange réactionnel est de 1:3 à 1:200 dans la seconde étape de réaction.

13. Procédé selon l'une ou plusieurs quelconques des revendications 8 à 12, caractérisé en ce que l'on utilise comme composés organiques du phosphore (III) des phosphines ou des phosphites aliphatiques, aromatiques ou mélangées aliphatiques-aromatiques.

14. Procédé selon la revendications 13, caractérisé en ce que le composé organique du phosphore (III) est une triarylphosphine et le rapport molaire du rhodium au phosphore dans le mélange réactionnel est de 1:50 à 1:150.

15. Procédé selon la revendication 14, caractérisé en ce que la triarylphosphine est la triphénylphosphine.

16. Procédé selon la revendication 13, caractérisé en ce que le composé organique du phosphore (III) est une trialkylphosphine et le rapport molaire du rhodium au phosphore dans le mélange réactionnel est de 1:3 à 1:20.

17. Procédé selon la revendication 16, caractérisé en ce que la trialkylphosphine est la trilaurylphosphine.
